# EUROPEAN PATENT SPECIFICATION

(11) **EP 4 296 356 B1**
(45) Date of publication and mention of the grant of the patent: **02.09.2026**
(21) Application number: 22926310.8
(22) Date of filing: 25.05.2022
(51) Int. Cl.: C12N 15/113, C12N 15/10, A61K 31/7105, A61P 31/16, A61P 31/14, A61P 11/00, A61P 29/00, A61K 36/481, A61K 36/634

(54) **FORSYTHIA SUSPENSA AND RADIX ASTRAGALI COMPOUND TRADITIONAL CHINESE MEDICINE DECOCTION-DERIVED MICRO RIBONUCLEIC ACID, AND PREPARATION METHOD THEREFOR AND USE THEREOF**
MIKRORNAS AUS FORSYTHIAE FRUCTUS-ASTRAGALI RADIX VERBINDUNG DER TRADITIONELLEN CHINESISCHEN MEDIZIN, VERFAHREN ZU IHRER HERSTELLUNG UND IHRE VERWENDUNG
MICRO-ACIDE RIBONUCLÉIQUE DÉRIVÉ D'UNE DÉCOCTION DE LA MÉDECINE CHINOISE TRADITIONNELLE À BASE DE FORSYTHIA SUSPENSA ET DE RADIX ASTRAGALI, SON PROCÉDÉ DE PRÉPARATION ET SON UTILISATION

(30) Priority: 21.04.2022 CN 202210424718
(43) Date of publication of application: 27.12.2023
(73) Proprietor: Nantong University, Nantong, Jiangsu 226019 (CN)
(72) Inventor: GU, Xiaosong, Nantong, Jiangsu 226019 (CN); MAO, Susu, Nantong, Jiangsu 226019 (CN); GONG, Leilei, Nantong, Jiangsu 226019 (CN); YANG, Xiaoming, Nantong, Jiangsu 226019 (CN); WANG, Xinghui, Nantong, Jiangsu 226019 (CN); SUN, Hualin, Nantong, Jiangsu 226019 (CN); XU, Lai, Nantong, Jiangsu 226019 (CN)
(74) Representative: Lorenz Seidler Gossel Part. mbB
(86) International application number: PCT/CN2022/094829
(87) International publication number: WO 2023/201836

(56) References cited:
- EP-A1- 2 886 652
- WO-A1-2020/124525
- CN-A- 101 076 592
- CN-A- 105 903 035
- CN-A- 111 249 299
- CN-A- 111 249 390
- US-A1- 2015 291 962
- ZHOU LI-KUN ET AL: "Absorbed plant MIR2911 in honeysuckle decoction inhibits SARS-CoV-2 replication and accelerates the negative conversion of infected patients", CELL DISCOVERY, vol. 6, no. 1, 5 August 2020 (2020-08-05), GB, XP093254381, ISSN: 2056-5968, Retrieved from the Internet <URL:https://www.nature.com/articles/s41421-020-00197-3> DOI: 10.1038/s41421-020-00197-3
- KHAN SHAZIA ET AL: "Identification and the potential involvement of miRNAs in the regulation of artemisinin biosynthesis in A. annua", SCIENTIFIC REPORTS, vol. 10, no. 1, 12 August 2020 (2020-08-12), US, XP093285466, ISSN: 2045-2322, Retrieved from the Internet <URL:https://www.nature.com/articles/s41598-020-69707-3.pdf> DOI: 10.1038/s41598-020-69707-3
- KHAN SHAZIA ET AL: "Identification and the potential involvement of miRNAs in the regulation of artemisinin biosynthesis in A. annua: supplemental data", SCIENTIFIC REPORTS, vol. 10, 12 August 2020 (2020-08-12), US, XP093285777, ISSN: 2045-2322, Retrieved from the Internet <URL:https://www.nature.com/articles/s41598-020-69707-3#Sec17> DOI: 10.1038/s41598-020-69707-3
- MOTIEGHADER HABIB ET AL: "Drug repurposing for coronavirus (SARS-CoV-2) based on gene co-expression network analysis", SCIENTIFIC REPORTS, vol. 11, no. 1, 8 November 2021 (2021-11-08), US, XP093285678, ISSN: 2045-2322, Retrieved from the Internet <URL:https://www.nature.com/articles/s41598-021-01410-3.pdf> DOI: 10.1038/s41598-021-01410-3
- LIU JUNCHENG ET AL: "Soybean-derived miRNAs specifically inhibit proliferation and stimulate apoptosis of human colonic Caco-2 cancer cells but not normal mucosal cells in culture", GENOMICS, ACADEMIC PRESS, SAN DIEGO, US, vol. 112, no. 5, 11 May 2020 (2020-05-11), pages 2949 - 2958, XP086211464, ISSN: 0888-7543, [retrieved on 20200511], DOI: 10.1016/J.YGENO.2020.05.011
- DATABASE NUCLEOTIDE ANONYMOUS : "Arabidopsis thaliana microRNA MIR159", XP093106476, retrieved from NCBI
- YANG XIAOMING, ZHANG YU; WANG XINGHUI; XU LINGCHI; XU LAI; GU XIAOSON: "Clinical Efficacy Analysis of Qiaoqi Granule Treating Influenza", MEDICAL JOURNAL OF COMMUNICATION, vol. 35, no. 1, 20 February 2021 (2021-02-20), pages 51 - 53, XP093106499, ISSN: 1006-2440, DOI: 10.19767/j.cnki.32-1412.2021.01.014
- Z HOU, ZHEN ET AL.: "Honeysuckle-encoded atypical microRNA2911 directly targets influenza A viruses", CELL RES, vol. 25, no. 1, 7 October 2014 (2014-10-07), pages 39 - 49, XP055247995, ISSN: 1748-7838, DOI: 10.1038/cr.2014.130

## Description

### TECHNICAL FIELD

The present disclosure belongs to the technical field of biological medicines, and specifically relates to microRNAs derived from a *Forsythiae Fructus-Astragali Radix* compound traditional Chinese medicine decoction as well as a preparation method and a use thereof.

### BACKGROUND

Influenza (flu for short) is an acute respiratory disease commonly suffered by mammals and poultry, is caused by influenza virus, has strong infectivity and high transmission speed, generally causes simple respiratory infection including cough, fever, myalgia, chill or sweating and other clinical symptoms, which can keep uncomfortable feeling for 2 days to 8 days, and generally causes rapid disease. Some patients, especially the elderly, young children and patients with other chronic diseases, are prone to viral or secondary pneumonia, even accompanied by respiratory difficulties and multiple organ failure. Anti-influenza medication is a common approach for resisting influenza at present, and the prescription is generally determined according to the infection degree of a patient and the physical condition of the patient. Although influenza virus is one of the most deeply studied pathogens, the existing control and treatment schemes need to be continuously improved due to the extreme variability of influenza virus, and the development of new drugs for viral influenza is a great demand of both the current society and the civil health.

The novel coronavirus (nCoV for short) is a novel virus widely spread in recent two years, is mainly spread through a respiratory pathway in a droplet form, and recently, the novel coronavirus is further found to be spread through aerosol and article contact. The novel coronavirus has extremely strong infectivity and extremely high spreading speed, and clinical symptoms are mainly as shown in follows: fever, fatigue, dry cough, olfactory deficits/disorders, severe cases may be accompanied by severe pneumonia with dyspnea, tachypnea and hypoxemia and may cause other complications. Because there is no specific therapeutic drug aiming at the new coronavirus at present, the coronavirus can be spread on a large scale all over the world. Therefore, in view of the medicine requirements for epidemic prevention and control, researching and developing a new medicine specific to the nCoV is an important means for dealing with the nCoV and guaranteeing the life safety of people.

MicroRNAs (miRNAs for short) are a micro endogenous non-coding RNAs. Typically, the miRNA coding gene is transcribed by RNA polymerase II and produces a primary transcript, that is processed by RNase III endonuclease Drosha and Dicer into microRNAs of approximately 21 nucleotides. MiRNA mediates post-transcriptional gene silencing through the binding to coding regions or 3 'and 5' non-translated regions of target mRNA, which plays an important role in various physiological and pathological processes, and has wide application prospects in the medical field.

In recent years, the related research of plant miRNA is rapidly developed, the biological function of the plant miRNA is mainly researched through an up-regulation/down-regulation strategy, and a plurality of researches show that the plant miRNA plays an important role in the aspects of photosynthesis, nutrition homeostasis, growth and development, hormone signal transduction, stress response and the like; in addition, plant miRNAs have been shown to regulate animal gene expression as endogenous mirnas. A great number of literatures report that traditional Chinese medicines play an active role in the process of preventing and treating influenza and novel coronavirus pneumonia. However, the specific active ingredient is still unclear. There have been literatures report that the traditional Chinese medicine contains miRNA, but there is still little understanding on the kind of stable miRNAs exist in traditional Chinese medicine decoction, and the functions these miRNAs will play when entering the animal bodies.

Related prior art is disclosed, for example, in EP2886652, in Zhou et al, 2020. Cell Discov, 6, 54, in CN105903035, in Khan et al, 2020. Sci Rep, 10, 13614, in Khan et al, 2020. Sci Rep, 10, 13614, supplemental data, in WO2020124525, in MotiGhader et al, 2011. Sci Rep, 11, 21872, or in Liu et al, 2020. Genomics, 112, 2949-2958.

### SUMMARY

In view of the technical deficiencies in the prior art, the present disclosure provides microRNAs derived from a *Forsythiae Fructus-Astragali Radix* compound traditional Chinese medicine decoction, and proves through experimental testings that the microRNAs QQ_159 from the *Forsythiae Fructus-Astragali Radix* compound traditional Chinese medicine decoction has certain inhibiting effect and treating effect on viral influenza and pneumonia caused by novel coronavirus infection.

The present disclosure is achieved through the following technical solutions.

Provided is microRNAs derived from a Forsythiae Fructus-Astragali Radix compound traditional Chinese medicine decoction. The microRNAs are QQ_159, and a a nucleotide sequence of the QQ_159 is as shown in SEQ ID NO.14. Alternative sequences not comprised by the claimed invention but nevertheless described herein are selected from novel_mir7, novel_mir33, novel_mir35, novel_mir10, novel_mir20, novel_mir5, novel_mir36, novel_mir28, novel_mir31, ppt-miR894, novel_mir32, novel_mir21, pab-miR3711, or bdi-miR159a-3p. A nucleotide sequence of the novel_mir7 is as shown in SEQ ID NO.1; a nucleotide sequence of the novel_mir33 is as shown in SEQ ID NO.2; a nucleotide sequence of the novel_mir35 is as shown in SEQ ID NO.3; a nucleotide sequence of the novel_mir10 is as shown in SEQ ID NO.4; a nucleotide sequence of the novel_mir20 is as shown in SEQ ID NO.5; a nucleotide sequence of the novel_mir5 is as shown in SEQ ID NO.6; a nucleotide sequence of the novel_mir36 is as shown in SEQ ID NO.7; a nucleotide sequence of the novel_mir28 is as shown in SEQ ID NO.8; a nucleotide sequence of the novel_mir31 is as shown in SEQ ID NO.9; a nucleotide sequence of the ppt-miR894 is as shown in SEQ ID NO.10; a nucleotide sequence of the novel_mir32 is as shown in SEQ ID NO.11; a nucleotide sequence of the novel_mir21 is as shown in SEQ ID NO.12; a nucleotide sequence of the pab-miR3711 is as shown in SEQ ID NO.13; and a nucleotide sequence of the bdi-miR159a-3p is as shown in SEQ ID NO.15. The present invention pertains to a microRNA derived from the Forsythiae Fructus-Astragali Radix compound traditional Chinese medicine decoction for use in treating viral influenza selected from Victoria influenza B or H5N1 avian influenza, or viral pneumonia caused by SARS-CoV-2 virus, wherein the microRNA is QQ_159 and the nucleotide sequence of the QQ_159 is as shown in SEQ ID NO.14.

Preferably, the microRNAs are QQ_159, and comprises artificially synthesized QQ_159, plant QQ_159 and a precursor form of QQ_159 or a mature form of QQ_159.

Also described, but not part of the claimed invention, is a method for preparing the microRNAs derived from a *Forsythiae Fructus-Astragali Radix* compound traditional Chinese medicine decoction includes the following steps.

In step 1), 300 mL of the Forsythiae Fructus-Astragali Radix compound traditional Chinese medicine decoction are divided and filled respectively into 50 mL enzyme removing centrifuge tubes; impurities are removed by centrifugation at 2000 rpm; 10 mL of supernatant are sucked from each tube and placed on ice, 30 mL of TRIzol are added at the radio of 3 times the volume of the decoction, kept at the room temperature for 10 minutes after shaking uniformly with force; 6 mL of chloroform are subsequently added, shaked uniformly with force, and kept for 10 minutes.

In Step 2), centrifugation is carried out at 10000 g and 4 °C for 10minutes, and supernatant is collected.

In Step 3), isopropanol with the same volume as that of the mixture is added to precipitate for 1 hour at the temperature of 20 °C, and then the precipitate is centrifuged at 12000 g for 15 minutes.

In Step 4), the supernatant is carefully discarded, 5 mL of 75% ethanol is added to wash the precipitate, and the precipitate is centrifuged at 12000 g for 5minutes at 4 °C.

In Step 5), after centrifugation, the ethanol is carefully removed, the precipitate is left, after the precipitate is dried, 200 µL of 65 °C DEPC-treated water is added for dissolving, and the RNA concentration is determined.

In Step 6), the miRNAs are purified with a commercial miRNA isolation kit, 100 µg of RNAs pass through each column, and the miRNAs in each tube are dissolved by 60 µL of DEPC-treated water.

In Step7), the concentration of miRNAs is determined, freeze-dried for 8 hours, and stored at -80 °C.

Provided, but not part of the claimed invention, is a use of the microRNAs QQ_159 derived from the *Forsythiae Fructus-Astragali Radix* compound traditional Chinese medicine decoction in a preparation of a medicament for inhibiting the replication of influenza virus.

Provided, but not part of the claimed invention, is a use of the microRNAs QQ_159 derived from the *Forsythiae Fructus-Astragali Radix* compound traditional Chinese medicine decoction in a preparation of a medicament for treating viral influenza.

The influenza comprises Victoria influenza B or HSN1avian influenza.

Provided, but not part of the claimed invention, is a use of the microRNAs QQ_159 derived from the *Forsythiae Fructus-Astragali Radix* compound traditional Chinese medicine decoction in a preparation of a medicament for inhibiting the replication of SARS-CoV-2 virus.

Provided, but not part of the claimed invention, is a use of the microRNAs QQ_159 derived from the *Forsythiae Fructus-Astragali Radix* compound traditional Chinese medicine decoction in a preparation of a medicament for treating viral pneumonia caused by SARS-CoV-2 virus.

Also described, but not part of the claimed invention, is a pharmaceutical composition for inhibiting influenza virus replication and/or treating influenza includes the microRNAs QQ_159, and a pharmaceutically acceptable carrier thereof.

Also described, but not part of the claimed invention, is a pharmaceutical composition for inhibiting the replication of SARS-CoV-2 virus and/or treating viral pneumonia caused by SARS-CoV-2 virus includes the microRNAs QQ_159, and a pharmaceutically acceptable carrier thereof.

Also described, but not part of the claimed invention, is a is a method for inhibiting replication of Victoria B influenza virus for non-therapeutic purposes in vitro, the above-mentioned microRNA QQ_159 is contacted with a cell infected with influenza B influenza virus.

The present disclosure has the following beneficial effects.

The present disclosure provides an experimental testing method for extracting microRNAs from a traditional Chinese medicine decoction, and 15 kinds of microRNAs stably exist in a *Forsythiae Fructus-Astragali Radix* compound traditional Chinese medicine decoction are extracted and identified by the method for the first time. It is demonstrated that the microRNAs QQ_159 have a certain inhibitory effect and therapeutic effect on pneumonia caused by viral influenza and novel coronavirus infection. The present disclosure not only obtains natural antiviral effective components of the traditional Chinese medicine, but also provides a possible nucleic acid medicine for clinically treating influenza or pneumonia caused by novel coronavirus infection.

### BRIEF DESCRIPTION OF THE DRAWINGS

FIG. 1 illustrates the inhibition effect of the microRNAs QQ_159 on the influenza B virus load tested by the real-time PCR in Example 2.
FIG. 2 illustrates the effect of the microRNAs QQ_159 on body weight of mice infected with avian influenza H5N1 virus in Example 3.
FIG. 3 illustrates the effect of the microRNAs QQ_159 on the survival rate of mice infected with avian influenza H5N1 virus in Example 3.
FIG. 4 illustrates the inhibition effect of the microRNAs QQ_159 on lung inflammation in mice infected with avian influenza H5N1 through the HE staining test in Example 3.
FIG. 5 illustrates the inhibition effect of the microRNAs QQ_159 on the pulmonary viral load of mice infected with avian influenza H5N1 on days 3 (A) and days 5 (B) tested by the real-time PCR in Example 3.
FIG. 6 illustrates the inhibition effect of the microRNAs QQ_159 on lung inflammation in mice infected with SARS-CoV-2 virus through the HE staining detection in Example 4.

### DETAILED DESCRIPTION OF THE EMBODIMENTS

In order to make the technical problems, technical solutions and advantages of the present disclosure more distinct, the following detailed description and specific embodiments are described with reference to the accompanying drawings.

### Example 1: Extraction of microRNAs from a Forsythiae Fructus-Astragali Radix compound traditional Chinese medicine decoction

(1) 300 mL of a Forsythiae Fructus-Astragali Radix compound traditional Chinese medicine (Lonicerae Japonicae Flos, Forsythiae Fructus, Scutellariae Radix, Artemisiae Annuae Herba, raw Astragali Radix, roasted Atractylodis Macrocephalae Rhizoma, Agastache rugosa, Saposhnikoviae Radix, Ophiopogon japonicus, Glycyrrhiza uralensis) decoction are respectively divided and filled into 50 mL enzyme-removing centrifuge tubes, and impurities are removed by centrifugation at 2000 rpm; 10 mL of supernatant are sucked from each tube and placed on ice; 30 mL of TRIzol are added at the radio of 3 times the volume of the decoction, kept at the room temperature for 10minutes after shaking uniformly with force, 6 mL of chloroform are subsequently added, shaked uniformly with force, and kept for 10 minutes.
(2) Centrifugation is carried out at 10000 g and 4 °C for 10 minutes, and the supernatant is collected.
(3) Isopropanol with the same volume as that of the mixture is added to precipitate for 1 hour (-20 °C), and then the precipitate is centrifuged at 12000 g for 15 minutes.
(4) The supernatant is carefully discarded, 5 mL of 75% ethanol is added to wash the precipitate, and the precipitate is centrifuged at 12000 g for 5minutes at 4 °C.
(5) After centrifugation, the ethanol is carefully removed, the precipitate is left, after the precipitate is dried, and 200 µL of 65 °C DEPC-treated water is added to dissolve the precipitate, and the RNA concentration is determined.
(6) The miRNAs are purified by a commercial miRNA isolation kit (100. µg of RNAs pass through each column) and dissolved in each tube by 60 µL DEPC-treated water.
(7) The concentration of miRNAs is determined, freeze-dried for 8 hours, and stored at -80 °C.
(8) The miRNAs are sequenced by BGISEQ-500 technique, and the results are as shown in Table 1 below.

**TABLE 1 Sequence list of microRNAs derived from a Forsythiae Fructus-Astragali Radix compound traditional Chinese medicine decoction**

| miRNA id | SEQ ID | Sequence (5'-3') |
|---|---|---|
| novel_mir7 | 1 | CCAGCACUAAUGCACCGGAUCCCAUCAG |
| novel_mir33 | 2 | UGGGAAGUCCUCGUGUUGUACCCCU |
| novel_mir35 | 3 | GCCUUCGAUGUCGGCUCUUCCUAUCAUU |
| novel_mir10 | 4 | UCGUCCAGCGGUUAGGAUAUCUGGCUUUC |
| novel_mir20 | 5 | GCGGAUCUUGGUGGUAGUAGCAAA |
| novel_mir5 | 6 | CUCGGGAAAAGGAUUGGCUCUGAGGGCUGG |
| nove_mir36 | 7 | ACACAUGCAAGUCGAACGUUGUUUU |
| novel_mir28 | 8 | GUGUGCACCGGUCGUCUCGUCCCUUCUG |
| novel_mir31 | 9 | UCUGGGUGGUGUAGUUGGUUAUCA |
| ppt-miR894 | 10 | CGUUUCACGUCGGGUUCACC |
| novel_mir32 | 11 | GUGGUUAGGACAUCGUCUUUUCA |
| novel_mir21 | 12 | GUCUGUAGUUCGAUCCUGCAUGGGGG |
| pab-miR3711 | 13 | UGGCGCUAGAAGGAGGGCCU |
| QQ_159 | 14 | UUUGGAUUGAAGGGAGCUCUA |
| bdi-miR159a-3p | 15 | CUUGGAUUGAAGGGAGCUCU |

### Example 2: Determination of the effect of QQ_159 on influenza B virus load in Madin Darby dog kidney (MDCK) cells

(1) MDCK cells are cultured in 24-well culture plates.
(2) 10 nM, 50 nM or 100 nM QQ_159a are transfected into MDCK cells respectively by the commercial transfection reagent riboFECTTM CP, while the nonsense sequence with the same concentration is transfected into MDCK cells as a control.
(3) The above-mentioned cells are infected with Victoria B influenza virus.
(4) After infecting the virus for 24 hours, the supernatant is collected and centrifuged, and cells are merged, RNAs are extracted, and the virus load is detected by real-time PCR.

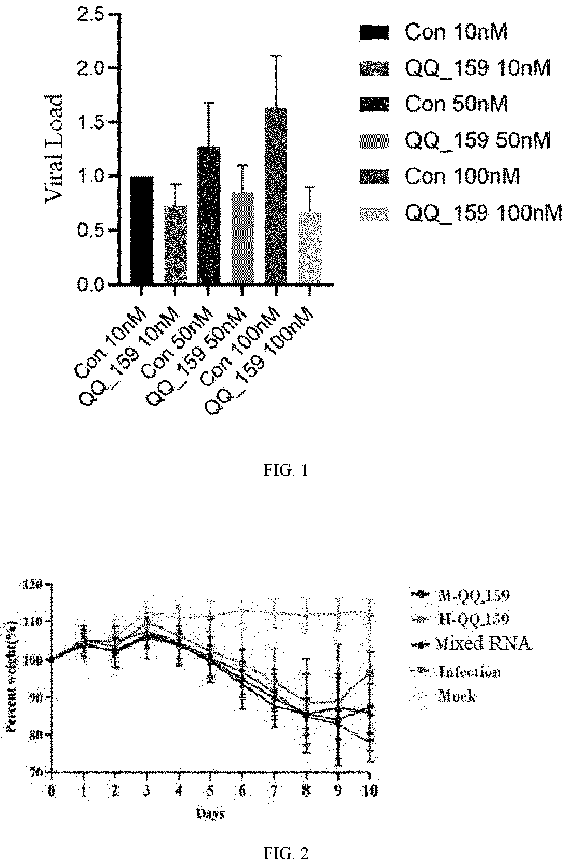

The results are as illustrated in FIG. 1, and 100 nM QQ _159 can significantly inhibit the viral load of Victoria B influenza virus in MDCK cells compared with the control group.

### Example 3 Determination of the therapeutic Effect of QQ_159 on mice infected with HSN1 subtype avian influenza virus

(1) Medicine are injected intraperitoneally to 6-week-old BALB/C female mice 2 hours after infected with HSN1 subtype avian virus. The animal groups and the drug dosages are as shown in Table 2 below.

**TABLE 2 QQ_159 avian influenza resistant H5N1 animal experimental testing groups and medicine treatment**

| Groups | | Dosages of virus attack | Administrati on dosages | Administration mode | Administrati on times | Numb er of mice |
|---|---|---|---|---|---|---|
| Group 1 | M-QQ_159 | 30 LD50/50 µL | 120 pmol/per mouse/day 0.1 mL | Intraperitoneal injection (DEPC-treated waterdilution QQ 159) | 5 | 16 |
| Group 2 | H-QQ_159 | 30 LD50/50 µL | 360 pmol/per mouse/day 0.1 mL | Intraperitoneal injection (DEPC-treated waterdilution QQ 159) | 5 | 16 |
| Group 3 | Mixed RNAGro up | 30 LD50/50 µL | 270 µg/per mouse/day 0.1 mL | Intraperitoneal injection (DEPC-treated waterdilution mixed RNA) | 5 | 16 |
| Group 4 | Infection Control Group | 30 LD50/50 µL | DEPC-treated water 0.1 mL | Intraperitoneal injection (DEPC-treated water) | 5 | 16 |
| Group 5 | Normal control group | DEPC-treated water 50µL | DEPC-treated water 0.1 mL | Intraperitoneal injection (DEPC-treated water) | 5 | 16 |

In Table 2: the mixed RNAs are mixed RNAs extracted from a *Forsythiae Fructus-Astragali Radix* compound traditional Chinese medicine decoction in a gathering way.

(2) The administration is continued for 5 days, and the change in body weights is observed and recorded every day (the mice are euthanized as death when the body weights are decreased by more than 30%).

(3) Lung tissues are taken on the third and fifth days after the virus attack respectively, and the inflammation condition of the lung tissues is detected by HE staining.

(4) Lung tissue viral load is detected through real-time PCR.

(5) Animals are housed until day 10 and the survival rate is observed.

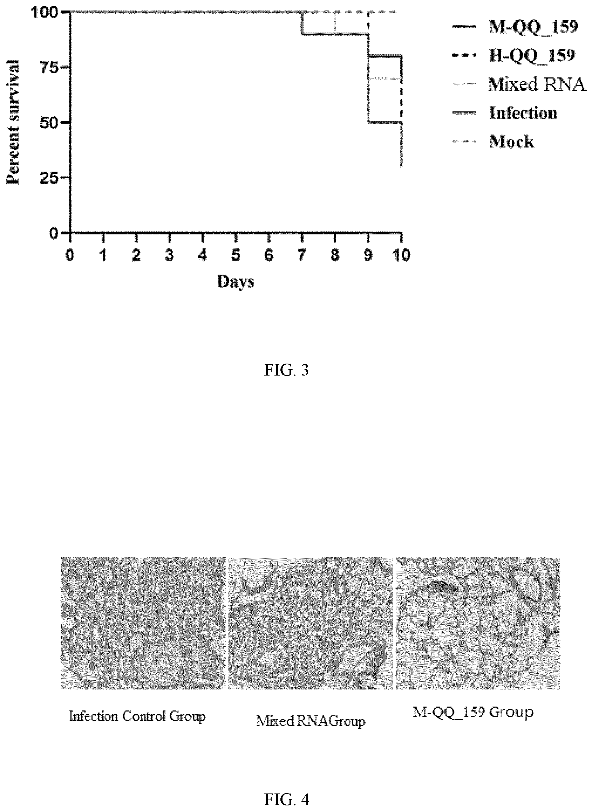

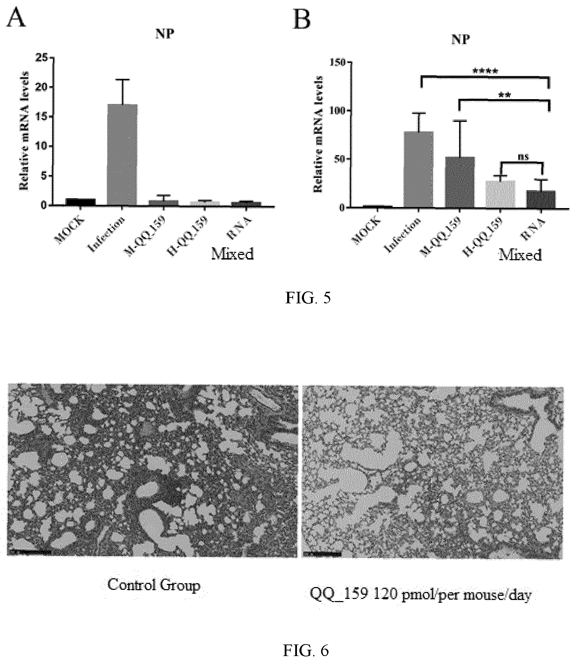

The result is that: in terms of change of the body weight, as illustrated in FIG. 2, the body weight changes of medium concentration QQ_159 (120 pmol/per mouse/day, M-QQ_159), high concentration QQ_159 (360 pmol/per mouse/day, H-QQ_159), and mixed RNA group at 7 days after virus attack (D8 is illustrated in FIG. 2) enter plateau, but the infected control group still have a tendency to decrease; the rate of change in body weight is slightly higher in the high concentration QQ_159 group than those in the mixed RNA group and the medium concentration QQ_159. In terms of survival rate, as illustrated in FIG. 3, the survival rate of the medium-concentration QQ_159 group and the mixed RNA group is 70%, the survival rate of the high-concentration QQ_159 group is 50%, and the survival rate of the infected group is merely 30%. As illustrated in FIG. 4, HE results show that lung inflammation is significantly reduced in the mixed RNA group and medium-concentration QQ_159 mice, and better inhibitory effect is exhibited in the medium-concentration QQ_159 than the infection control group. As illustrated in FIG. 5A, the medium-concentration QQ_159 group, the high-concentration QQ_159 group, and the mixed RNA group are all effective in inhibiting viral replication on the third day after infection; as illustrated in FIG. 5B, on the fifth day after infection, the medium concentration QQ_159 group exhibits a significantly reduced effect of inhibiting viral replication, while the high concentration QQ_159 group and the mixed RNA group still exhibit a better effect of inhibiting viral replication.

The experimental testing shows that the artificially synthesized QQ _159 and the mixed RNA extracted from a *Forsythiae Fructus-Astragali Radix* compound traditional Chinese medicine decoction have certain treatment effect on the HSN1 avian influenza virus, which is represented by delaying weight loss, reducing mortality and reducing lung inflammation.

### Example 4 Research of the inhibition effect of QQ_159 on pneumonia caused by SARS-CoV-2 virus

(1) The hACE2 mice at the age of 6 to 8 weeks are infected with SARS-CoV-2 at 10⁵TCID₅₀/ml in form of nasal drops.
(2) Mice are administered by intraperitoneal (ip) injection for 5 days. The mice are administered after being infected for 2 hours on the first day. The experimental testing groups and the dosages are as shown in Table 3 below.

**TABLE 3 SARS-CoV-2 infection experimental testing of hACE2 transgenic mice**

| Group | Administration dosages | Administrat ion volume | Administra tion mode | Time of virus attack | Times |
|---|---|---|---|---|---|
| 1 | 60 pmol/per mouse/day | 100 µL/per mouse | ip | Administration after infection of 2h | 5 |
| 2 | 120 pmol/per mouse /day | 100 µL/per mouse | ip | Administration after infection of 2h | 5 |
| 3 | 180 pmol/per mouse /day | 100 µL/per mouse | ip | Administration after infection of 2h | 5 |
| Control Group | - | 100 µL/per mouse | ip | Virus attack at day 0 | 5 |

(3) Body weight and symptoms are monitored on days 0, 1, 2, 3, 4, 5.
(4) Mice are sacrificed on the fifth day after infection and lung tissues are collected.
(5) Lung pathology is detected by HE staining

As illustrated in FIG. 6, 120 pmol/per mouse/day injection of QQ_159 exhibits a certain inhibitory effect on the lung tissue inflammation of hACE2 transgenic mice infected with SARS-CoV-2, indicating that QQ_159 exhibits a certain inhibitory effect on the mouse pneumonia caused by SARS-CoV-2 infection.

## Claims

1. A microRNA derived from the *Forsythiae Fructus-Astragali Radix* compound traditional Chinese medicine decoction for use in treating viral influenza selected from Victoria influenza B or H5N1 avian influenza, or viral pneumonia caused by SARS-CoV-2 virus, wherein the microRNA is QQ_159 and the nucleotide sequence of the QQ_159 is as shown in SEQ ID NO. 14.

## Patentansprüche

1. MicroRNA, abgeleitet aus dem Abkochungsdekokt der zusammengesetzten traditionellen chinesischen Medizin aus *Forsythiae Fructus* und *Astragali Radix,* zur Verwendung bei der Behandlung von viraler Influenza, ausgewählt aus Victoria-Influenza B oder H5N1-Vogelgrippe, oder viraler Pneumonie, verursacht durch das SARS-CoV-2-Virus, wobei die microRNA QQ_159 ist und die Nukleotidsequenz der QQ_159 wie in SEQ ID NO.14 dargestellt ist.

## Revendications

1. MicroARN dérivé de la décoction de médecine traditionnelle chinoise composée de *Forsythiae Fructus et d'Astragali Radix,* pour une utilisation dans le traitement de la grippe virale choisie parmi la grippe B Victoria ou la grippe aviaire H5N1, ou de la pneumonie virale causée par le virus SARS-CoV-2, dans lequel le microARN est QQ_159 et la séquence nucléotidique de QQ_159 est telle que représentée dans SEQ ID NO.14.
